Europäisches Patentamt

European Patent Office

Office européen des brevets

(11) Publication number: 0 377 943 A1

# (12) EUROPEAN PATENT APPLICATION

(21) Application number: 89310245.9

(22) Date of filing: 06.10.89

(51) Int. Cl.⁵: A61M 25/00, A61B 5/03

(30) Priority: 09.01.89 US 295074

(43) Date of publication of application:
18.07.90 Bulletin 90/29

(84) Designated Contracting States:
DE FR GB IT NL SE

(71) Applicant: THE BOC GROUP, INC.
575 Mountain Avenue
Murray Hill New Jersey 07974(US)

(72) Inventor: Schaberg, Richard R.
220 Eva Street
Ventura, CA 93003(US)
Inventor: Kodama, Roy K.
1491 E. Uppingham Drive
Thousand Oaks, CA 91360(US)
Inventor: Cole, James E.
891 Via Arroyo
Ventura, CA 93003(US)

(74) Representative: Gough, Peter et al
c/o THE BOC GROUP PLC Patent Department
Chertsey Road
Windlesham Surrey GU20 6HJ(GB)

(54) Intrauterine catheter.

(57) An intrauterine catheter tip pressure transducer is disclosed constructed as to have outside dimensions extremely minute and having a substantially uniform outside diameter throughout its entire length. The transducer comprises an electronic sensor (40) of the etched diaphragm type, comprising a piezoresistive die or chip having dimensions of about .080 inches square and which is mounted in the distal end (18) of the catheter (10). One side of the transducer diaphragm is acted upon by the pressure within the uterus and the other side of the diaphragm is placed at a known reference pressure, preferably atmospheric pressure. A passage (28) is provided within the interior of the catheter (10) for applying the reference pressure to the transducer. The sensor (40) is directly mounted to an annular frame (20) at the distal end and surrounded by a cylindrical sleeve (30) that is crimped to the catheter tubing (12). Wiring is provided directly to the sensor (40) by ribbon cable (50) passing within the catheter (10) from its proximal end (14) to free ends (52) within the distal end (18) where the free ends (52) are directly bonded, by gold wedge bonding (58), to the pads (56) of the electronic sensor (40), thereby eliminating larger printed circuit boards or conductive tape to make that connection. Thus the overall catheter (10) may have its distal end about 3.3mm in outer diameter with the catheter tubing of about 3.0mm. By the preferred construction, the overall dimensions can be reduced so as to be used with standard sized introducers presently used for intrauterine pressure determinations.

FIG.3

## INTRAUTERINE CATHETER

This invention relates to a pressure sensing catheter and a method of making the same and, in particular, to an intrauterine catheter for sensing the amniotic fluid pressure within a woman during pregnancy.

In difficult or suspected difficult childbirth, it is important to monitor fetal activity such as fetal heart rate and intrauterine pressure as an indication of the overall well being of the fetus. As to the present means of monitoring such activity, monitors are available that measure the pressure within the uterus, detect uterine contractions or other pressure changes and also detect and monitor fetal heart rate.

Presently, such intrauterine pressure monitors are external or internal. Tacodynamometers can be used externally and which measure the duration and frequency of uterine contractions. Although such external monitoring is useful and convenient to utilize, it also suffers from difficulties in picking up and interpreting extraneous interference. The external interference such as motion and noise artifact increases as childbirth approaches and thus, external monitors of uterine contractions as well as fetal heart rate often have difficultly in obtaining accurate readings at the critical times that accuracy is needed.

Accordingly, internal fetal monitoring is more accurate since it is not subject to the outside extraneous interference as is external monitoring and gives a more stable reading of uterine contractions and pressures.

One very common means of internal uterine pressure monitoring is the fluid filled catheter tube that is inserted directly into the uterus. Much the same way that in-vivo arterial blood pressure is monitored, a pressure transducer is located external to the patient so as to measure the pressure in a column of fluid, one end of which is placed directly within the uterus and the other end is connected to the external transducer.

The fluid is displaced in the catheter by pressure changes in the uterus and those changes in fluid displacement are sensed by a diaphragm in the pressure transducer and converted to electrical signals to be supplied to a monitor.

Typically, the fluid filled catheter is introduced into the uterus by being threaded through an introducer or guide tube that in turn is positioned manually by the attending doctor. Upon placement of the introducer, the fluid filled catheter itself is fitted through the introducer where it is moved forward into the desired position adjacent the fetus.

Fluid filled catheters are more accurate in determining amniotic pressures since the catheter open end is directly placed at the location for which information is sought, however, such catheters also suffer from certain difficulties. They require a container of sterile liquid, valves, and can also be subject to motion artifact. In addition, problems arise by the presence of bubbles and the fluid filling procedures are relatively cumbersome to carry out.

Fairly recently, catheter tip pressure transducers have been developed wherein the pressure transducer itself is located in the catheter tip and thus directly measures and converts to electrical signals, the pressure within the uterus at the catheter tip. Typical devices utilize solid state piezoresistive crystals and which may be etched on one or both sides to form a flexible diaphragm in the electronic chip or die. A device of this type is shown and described in U.S. patent 4,726,232 of Koneval and such devices are readily manufacturable by, known methods. As used herein, the electronic monocrystalline silicon piezoresistive die as described in the aforementioned U.S. patent may be referred to as the electronic sensor for brevity. Such electronic sensors essentially are minute strain guages and the amount of flexing of the etched diaphragm is indicative of the differential pressure across that diaphragm. By placing one side of the diaphragm at a known, reference pressure, such as atmospheric pressure, one can measure the pressure that is applied to the other side of the diaphragm and which is mounted within the catheter tip so as to be acted upon by the pressure sought to be measured.

Catheter tip pressure transducer (CTPT's) have been used to measure intrauterine pressure for monitoring fetal activity and one such device is sold by Utah Medical Products under the trademark INTRAN.

One difficulty with the latter CTPT is that, due to its particular construction, the tip of the catheter is relatively large, i.e., 0.35 inches wide, particularly when considered against the catheter outside diameter of 0.160 inches.

Thus the catheter essentially has a large bulge at its end, the diameter increases over 200 percent. In order to utilize such a catheter, large diameter tubing is necessitated and integral stiffening wire. For the application as intrauterine catheter, a large dilation is required for catheter placement on the order of 2 cm. Obviously, a smaller overall catheter with a uniform sized tip is preferred, providing accuracy is not compromised. Of a particular disadvantage with the aforementioned prior art catheter, is that one of the recognized methods of introducing an intrauterine pressure catheter is by use of an introducer, as currently used with fluid-filled pressure catheters. Since many physicians are comfortable with the use of such introducers, they prefer to use that method of introduction.

Introducers typically used are of the size 14 French which corresponds to an outer diameter of 0.104 inches and therefore a catheter such as the aforedescribed catheter cannot be used since the catheter tip containing the pressure transducer is too large and is non-uniform with respect to the catheter tubing.

Other types of catheter tip pressure transducers are in use for blood pressure monitors, such as disclosed in U.S. Patent 4,722,348, however, again, the particular construction details prevent the reduction in cost sufficient to be utilized for the present purposes. Cantilever beam devices adaptable for use in a catheter tip include Schaberg et al U.S. Patent 4,545,389 and Millar U.S. Patents 3,748,623 and 3,724,274.

It is an aim of the present invention to provide a catheter tip intrauterine pressure transducer which is constructed to overcome the difficulties associated with the aforedescribed prior art.

According to one aspect of the present invention a catheter for measuring the pressure of a fluid within the body of a patient, said catheter having a distal end for insertion into the body and a proximal end which in use remains outside the body is characterised in that said distal end includes a chamber having an opening, an electronic sensor mounted in said chamber and responsive to differential pressures across two surfaces thereof, said electronic sensor being sealed across the opening such that one of said surfaces is acted upon by pressure within the chamber whilst the other surface is acted upon by pressure external to said catheter, a passage in the catheter for introducing a known pressure to said chamber, electrical conductors having first ends terminating in external connections at the proximal end of the catheter and second ends terminating at said distal end within the chamber, device wiring means directly connecting said electrode sensor to said second ends of the electrical conductors at said distal end and in that said catheter is substantially uniform in outer dimensions.

In particular, the catheter is comprised of a flexible tubing, preferably polyethyene, having a proximal end providing electrical connections and a distal end that includes a solid state electronic sensor mounted in a particular manner. The device is a known silicon chip strain gage type as shown and described in the aforementioned U.S. Patent 4,726,232 having dimensions of about .080 inches square.

The electronic sensor device is mounted such that the overall outer diameter of the distal tip of the catheter containing the electronic sensor is preferably no larger than 3.3mm or a size 10 French with a catheter tubing preferably of about 3.0mm or size 9 French. Accordingly, the catheter may be introduced into the human body by means of an introducer and is relatively uniform by having its distal tip diameter of approximately 10 percent larger than the catheter tubing outside diameter.

In mounting the electronic sensor at the distal end of the catheter, a special catheter tip is constructed wherein electrical wires are directly formed between the pads of the sensor and the free ends of ribbon cable that passes through the catheter from electrical connector located at the proximal end, thus greatly reducing the size requirements by eliminating any intermediate printed circuit boards or other connection means.

In addition, the outside diameter of the distal end of the catheter is reduced, with respect to the prior art, by affixing the electronic sensor directly to a frame such that one side of the electronic sensor is directly associated with the pressure sought to be sensed while the other side is acted upon by a known pressure within a chamber formed in the distal end.

That chamber is created by a thin cylindrical sleeve that surrounds the frame and which sleeve is crimped to the end of the flexible tubing. Various seals are made at surfaces covered by the sleeve and the sleeve itself may be typically as small as 3.3mm in outer diameter and crimped to a flexible tubing having an outside diameter of about 3.0mm, thus the outer dimension of the entire catheter does not vary by more than about ten percent, throughout its entire length. Due to the particular construction, the overall intrauterine catheter is reduced in size with respect to the prior art catheters, is sufficiently flexible without kinking, and can be introduced into the uterus by means of introducers of conventional size internal and external diameters.

According to a further aspect of the present invention a method of joining insulated electrical conductors to a silicon transducer sensor located in the distal end of a pressure measuring catheter is characterised by the steps of:

a) providing a flat planar surface in the distal end of said catheter adjacent the silicon sensor;

b) flattening the insulated ends of the electrical conductors to be connected to the silicon sensor to create oppositely disposed flattened surfaces;

c) affixing the flattened ends of the electrical conductors to the flat planar surface;

d) removing the insulation from the other flattened surface of the electrical conductors opposite the bonded surface;

e) bonding wires from said silicon sensor to the uninsulated flattened surface of the electrical conductors.

An embodiment of the invention will now be described, by way of example, reference being made to the Figures of the accompanying diagrammatic drawings in which:-

Figure 1 is a side view of an intrauterine catheter constructed in accordance with the present invention;

Figure 2 is a cross-sectional view of the catheter of Figure 1, rotated 90° with respect to Figure 1;

Figure 3 is an enlarged cross-sectional view, broken away, of the distal end of the catheter of Figure 1;

Figure 4 is a plan view of the internal parts of the distal end and showing the wiring connections to the electronic chip strain gage;

Figure 5 is an enlarged perspective view of the free ends of wires modified during the steps of directly connecting those free ends to the electronic sensor; and

Figure 6 is a schematic view showing the catheter of the present invention used with an introducer.

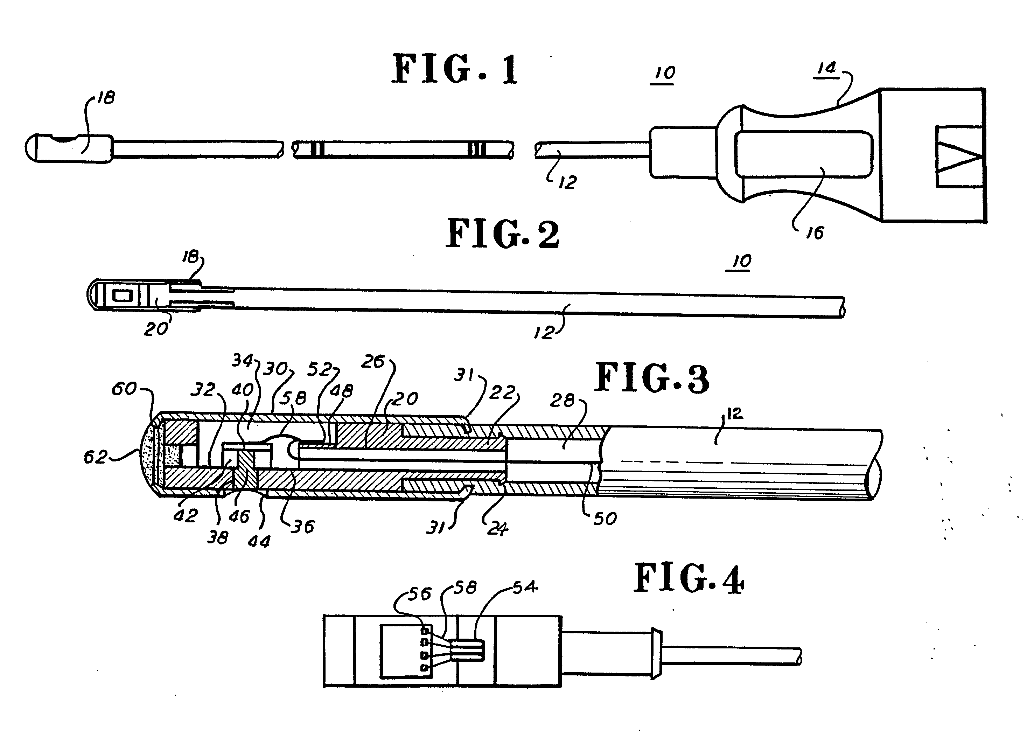

Turning first to Figure 1, there is shown a side view of an intrauterine pressure transducer catheter 10 constructed in accordance with the present invention.

In particular, the catheter 10 comprises flexible tubing 12, preferably of polypropylene or polyethylene. The material requires certain flexibility yet, obviously, must be bio-compatible with the body where pressures are being measured. Catheter 10 further includes a proximal end 14 that, in use, remains outside the body and has an electrical connector 16 for connection to a mating connector for wiring to a monitor to display the pressures being monitored. A distal end 18 is located at the other end of catheter 10 and is inserted into the body to be positioned at the very location therein where pressure determinations are desired.

Taking now, Figures 2 and 3, further details of the intrauterine catheter 10 can be seen wherein Fig 2 is a plan view, partially broken away and Figure 3 is an enlarged side cross-sectional view of the distal end 18 of catheter 10.

As can be seen, a tubular frame 20 is affixed to the end of flexible tubing 12. In the preferred embodiment, tubular frame 20 includes a reduced diameter cylindrical projecting stem 22 that is fitted within the internal diameter of flexible tubing 12 to affix tubular frame 20 thereto. As a further aid in insuring the proper affixation of tubular frame 20, at least one barb 24 is formed at or near the projecting end of projecting stem 22 and which firmly grips the internal surface of flexible tubing 12.

Tubular frame 20 includes an cylindrical bore 26 that communicates with the interior hollow opening 28 running through the length of flexible tubing 12. The tubular frame 20 is made of rigid material and can include stainless steel or a plastic such as polycarbonate.

A cylindrical sleeve 30 surrounds tubular frame 20 and is secured to the outer diameter of flexible tubing 12. That securing means is provided by crimping the open end 31 of cylindrical sleeve 30 about flexible tubing 12. Cylindrical sleeve 30 must be a material of sufficent hardness, yet bio-compatible with the human body, thus, a preferred material is austenitic stainless steel. Therefore, physical crimping is utilized in view of the difficulty of achieving a bond between austenitic steel and the preferred polyethylene material of flexible tubing 12.

As noted, the outer diameter of cylindrical sleeve 30 is only slightly larger than the outer diameter of flexible tubing 12; in the preferred embodiment, the outer diameter of cylindrical sleeve 30 may be about 3.3mm, being equivalent to a size 10 French, while the flexible tubing 12 has an outer diameter of about 3.0mm; or equivalent to a size 9 French. Thus the two outer diameters are substantually the same with an increase or difference in diameter being in the range of 10 percent.

A cut away area 32 is formed in tubular frame 20 and which forms a chamber 34 within the cylindrical sleeve 30. That cut away area 32 is formed such as to create a first flat area 36 and which has an aperture 38 therethrough.

A pressure transducer is mounted to the first flat area 36 and comprises a monocrystalline silicon piezoresistor sensor 40 anodically bonded to a borosilicate glass isolator 42. The sensor 40 is of a type commonly used in blood pressure transducers and specifically can be made as disclosed in U.S. Patent 4,726,232, it being noted that, in the present invention, such sensors can conventionally be made having dimensions of about .080 inches square.

As can be specifically seen in Figure 3, the sensor 40 has one surface thereof directly acted upon by the pressure in chamber 34 and its other surface is acted upon by pressures external to catheter 10 through the aperture 38 in tubular frame 20 and corresponding aperture 44 in cylindrical sleeve 30.

A fluid dielectric medium 46 fills aperture 38 and may be silicone gel to provide electrical and chemical isolation of sensor 40 yet transmit pressure of the amniotic fluid being measured external of catheter 10 within the uterus.

A second flat area 48 is formed in tubular frame 20 and is used to make electrical connections between ribbon cable 50 and sensor 40.

Although not shown in the Figures, ribbon cable 50 is connected at one end to the electrical connector 16 located at the proximal end 14 of catheter 10 and has its other free end 52 terminating within chamber 34 at the distal end 18 of catheter 10. Typically ribbon cable 50 may pass within the hollow opening 28 in flexible tubing 12 and is a multi-conductor cable covered by insulation, normally a four conductor cable where the individual conductors may have an outside diameter of about 0.005 inches.

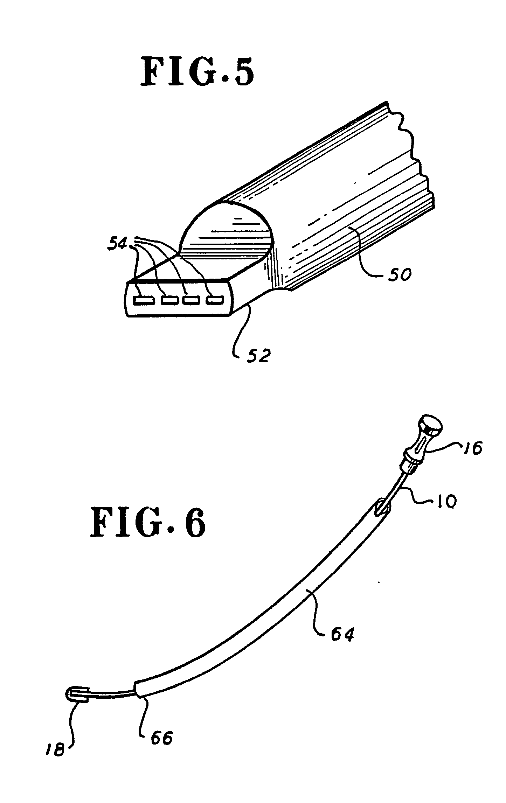

In the procedure for making electrical connection between free end 52 of ribbon cable 50 and sensor 40, the free end 52 of ribbon cable 50 is first flattened as shown in the perspective view of Figure 5. The amount of flattening needs to be sufficient to provide an upper surface of each conductor cable having a width of about 0.006 inches. Upon flattening of sufficient amount, the free end 52 is twisted backward and affixed to second flat area 48 by means such as an epoxy cement. Next, an instrument is used to scrape the upper insulation from the multi-conductor ribbon cable 50 such that all of the conductors have bare upper surfaces 54 (Figure 4).

Connection can now be accomplished directly from the conductor pads 56 of the sensor 40 to the bared, flattened upper surfaces 54 of free end 52 of ribbon cable 50 by means of a conventional gold wedge bonding machine to create a gold wedge bond 58 between the conductive pads 56 and upper surfaces 54 of the individual conductors of ribbon cable 50.

It is noted, and of great importance in achieving the extremely minute outside diameter of the distal end 18 of catheter 10 that the gold bonding be directly from sensor 40 to the conductors of ribbon cable 50 thus eliminating any intermediate circuit boards, conductive tape or the like that would necessitate increasing the size of distal end 18.

The distal end 60 of tubular frame 20 can be sealed by an epoxy plug 62 which also seals against cylindrical sleeve 30. Alternatively, cylindrical sleeve 30 may be deep drawn and have a closed end such that the epoxy plug 62 would not be necessary. Additional epoxy cement may be applied about the periphery of the aperture 44 of cylindrical sleeve 30 to seal therearound as well as between cylindrical sleeve 30 and the outer surface of tubular frame 20 to prevent pressure leaks or movement between those components.

Finally, in Figure 6 there is shown a perspective view of catheter 10 constructed in accordance of the present invention inserted through an introducer 64. As stated, in carrying out one of the methods of locating distal end 18 of catheter 10 at the desired location within the uterus, a physician manually locates the distal end 66 of introducer 64 at the entrance to the uterus and then guides the catheter 10 through the introducer 64 such that the distal end 18 reaches the desired position for monitoring the intrauterine pressure adjacent the fetus.

Conventional introducers now used with fluid-filled catheters have internal openings of about 0.144 inches or size 11 French. Thus, the catheter 10 of the present invention can be utilized with conventional sized introducers in accordance with present practices. The physician thereby obtains the flexibility and convenience of use with an introducer, yet obtains the additional accuracy and motion artifact-free performance of the catheter tip pressure transducer. As a further convenience, introducer 64 may be of the splittable type that is removed after placement of the catheter, by splitting the introducer longitudinally and removing it entirely.

## Claims

1. A catheter 10 for measuring the pressure of a fluid within the body of a patient, said catheter 10 having a distal end 18 for insertion into the body and a proximal end 14 which in use remains outside the body characterised in that said distal end 18 includes a chamber 34 having an opening 38, an electronic sensor 40 mounted in said chamber 34 and responsive to differential pressures across two surfaces thereof, said electronic sensor 40 being sealed across the opening 38 such that one of said surfaces is acted upon by pressure within the chamber 34 whilst the other surface is acted upon by pressure external to said catheter 10, a passage 28 in the catheter 10 for introducing a known pressure to said chamber 34, electrical conductors having first ends terminating in external connections at the proximal end 14 of the catheter 10 and second ends terminating at said distal end 18 within the chamber 34, device wiring means 58 directly connecting said electronic sensor 40 to said second ends of the electrical conductors at said distal end 18 and in that said catheter 10 is substantially uniform in outer dimensions.

2. A catheter as claimed in Claim 1 characterised in that said catheter 10 comprises a flexible polyethylene tubing 12 and said distal end 18 includes a thin walled cylindrical sleeve 30 crimped to said flexible polyethylene tubing 12.

3. A catheter as claimed in Claim 2 characterised in that the outer diameter of said thin walled cylindrical sleeve 30 is no more than about ten percent larger than the outer diameter of said flexible polyethylene, tubing 12.

4. A catheter as claimed in any one of Claims 1 to 3 characterised in that said other surface of said electronic sensor 40 is covered with an electrically non-conductive material 46 that transmits the pressure from within the body to said other side of said electronic sensor 40.

5. A catheter as claimed in Claims 1 to 4 characterised in that said electronic sensing device 40 is an etched diaphram silicon chip having dimensions of about .080inches square.

6. A catheter as claimed in any one of Claims 2 to 5, characterised in that a connector 16 is affixed to the proximal end 14of the tubing 12 for connection to an external monitor and in that a subular frame 20 is affixed to the distal end 18 of the tubing 12 and extends outwardly therefrom within the cylindrical sleeve 30, the frame 20 having an aperture 38 and having at least one planar surface 48, the frame 20 and sleeve 30 defining the chamber 34, the sleeve 30 having an aperture 44 in alignment with said aperture 38 in the frame, said electronic sensing device being in the form of a monocrystalline silicon transducer 40 affixed to the frame 20 and sealed to close the aligned aperture 38,44, means 28 for introducing a known pressure into said chamber 34, a first set of electrical conductors located within the tubing 12 having a first set of ends affixed to the connector 16 and a second set of ends 52 affixed to the at least one planar surface 48, and a second set of bonding conductors 58 electrically connected to said transducer 40 within the chamber 34 and directly to said second set of ends 52 to receive and transmit electrical signals indicative of pressure within the body.

7. A catheter as claimed in Claim 6 characterised in that said tubular frame 20 includes a reduced diameter cylindrical projecting stem 22 fitted within the inside diameter of said polymeric tubing 12, and said reduced diameter projecting stem 22 has at least one outwardly projecting barb 24 for adhering to said polymeric tubing 12.

8. A catheter as claimed in Claim 6 or 7 characterised in that said second set of ends of electrical conductors has flattened, planar surfaces 54 for connection to said second set of bonding conductors 58.

9. A catheter as claimed in Claim 8 characterised in that said second set of electrical bonding conductors 58 are wires sonic bonded to said second set of ends of said electrical conductors.

10. A catheter as claimed in Claim 9 characterised in that said second set of electrical bonding conductors 58are gold bonded conductors directly from said transducer 40 to said flattened, planar surfaces 54 of said second set of ends 52 of said electrical conductors.

11. A catheter as claimed in any one of Claims 6 to 10 characterised in that said at least one planar surface comprises at least two planar surfaces 36,48 formed in said tubular frame 20, and wherein said second set of ends 52of said electrical conductors are affixed to one of said planar surfaces 48 and said transducer 40is affixed to said tubular frame 20 on said other planar surface 36.

12. A method of joining insulated electrical conductors to a silicon transducer sensor 40 located in the distal end 18 of a pressure measuring catheter 10, characterised by the steps of:

a) providing a flat planar surface 48 in the distal end 18 of said catheter 10 adjacent the silicon sensor 40;

b) flattening the insulated ends of the electrical conductors to be connected to the silicon sensor 40 to create oppositely disposed flattened surfaces 54;

c) affixing the flattened ends 54 of the electrical conductors to the flat planar surface 48;

d) removing the insulation from the other flattened surface of the electrical conductors opposite the bonded surface;

e) bonding wires from said silicon sensor 40 to the uninsulated flattened surface of the electrical conductors.

13. A method as claimed in Claim 12 characterised in that said bonding step comprises utilizing a gold wedge bonding method to form a gold bond between the sensor 40 and said flattened surface 54 of said electrical conductors.

14. A method as claimed in Claim 12 characterised in that said bonding step comprises epoxy bonding said ends of the insulated wires to the flat planar surface.

FIG. 1
FIG. 2
FIG. 3
FIG. 4

# FIG.5

54

50

52

# FIG.6

16

10

64

18

66

EP 89310245.9

## DOCUMENTS CONSIDERED TO BE RELEVANT

| Category | Citation of document with indication, where appropriate, of relevant passages | Relevant to claim | CLASSIFICATION OF THE APPLICATION (Int Cl.⁵) |
|---|---|---|---|
| X | US - A - 4 785 822 (W.D. WALLACE) * Totality, especially fig. 1,2,4; column 5, line 46 - column 9, line 21 * | 1,4 | A 61 M 25/00 A 61 B 5/03 |
| Y | | 5 | |
| A | | 2,3,6 | |
| D,Y | US - A - 4 726 232 (D.J. KONEVAL) * Totality * | 5 | |
| Y | US - A - 4 023 562 (J. HYNECEK) * Totality * | 5 | |
| A | | 1 | |
| D,A | US - A - 4 722 348 (H. LIGTENBERG et al.) * Totality * | 1,4-6 | |
| A | EP - A1 - 0 263 190 (PPG HELLIGE B.V.) * Totality; especially column 2, line 35 - column 4, line 45 * | 1,4,6 | TECHNICAL FIELDS SEARCHED (Int Cl.⁵) A 61 B 5/00 A 61 M 23/00 A 61 M 25/00 |
| A | US - A - 4 274 423 (M. MIZUNO et al.) * Totality; especially fig. 3,6,7,13 * | 1,4-6 | |

The present search report has been drawn up for all claims

| Place of search | Date of completion of the search | Examiner |
|---|---|---|
| VIENNA | 26-04-1990 | LUDWIG |